# EUROPEAN PATENT SPECIFICATION

(11) **EP 3 517 619 B1**
(45) Date of publication and mention of the grant of the patent: **27.07.2022**
(21) Application number: 17853323.8
(22) Date of filing: 01.09.2017
(51) Int. Cl.: C12P 7/64, C12P 7/649, A61Q 19/00, A61K 8/37, A23L 29/00

(54) **METHOD FOR PREPARING FATTY ACID ETHYL ESTER**
VERFAHREN ZUR HERSTELLUNG VON FETTSÄUREETHYLESTERN
PROCÉDÉ DE PRÉPARATION D'ESTER ÉTHYLIQUE D'ACIDE GRAS

(30) Priority: 23.09.2016 KR 20160122417
(43) Date of publication of application: 31.07.2019
(73) Proprietor: CJ Cheiljedang Corporation, Seoul 04560 (KR)
(72) Inventor: LEE, Yunjeong, Seongnam-si Gyeonggi-do 13595 (KR); KIM, Mijung, Suwon-si Gyeonggi-do 16593 (KR); PARK, Seung Won, Yongin-si Gyeonggi-do 16894 (KR); LEE, Sang Bum, Seoul 08202 (KR); CHO, Seong Jun, Seoul 04971 (KR)
(74) Representative: Regimbeau
(86) International application number: PCT/KR2017/009625
(87) International publication number: WO 2018/056609

(56) References cited:
- EP-A1- 0 502 697
- EP-A1- 2 561 763
- EP-A1- 2 851 361
- EP-B1- 0 502 697
- WO-A1-2011/161702
- WO-A1-2016/136751
- WO-A2-2009/040676
- JP-A- 2013 032 439
- KR-A- 20110 069 421
- KR-A- 20150 013 257
- MENG, YONGHONG ET AL.: 'Two-step Synthesis of Fatty Acid Ethyl Ester from Soybean Oil Catalyzed by Yarrowia Lipolytica Lipase' BIOTECHNOLOGY FOR BIOFUELS vol. 4, 02 March 2011, pages 1 - 9, XP002656271
- Fidèle P Tchobo ET AL: "ENZYMATIC SYNTHESIS OF COCOA BUTTER EQUIVALENT THROUGH TRANSESTERIFICATION OF PENTADESMA BUTYRACEA BUTTER", JOURNAL OF FOOD LIPIDS, vol. 16, no. 4, 1 November 2009 (2009-11-01), pages 605-617, XP055660553, US ISSN: 1065-7258, DOI: 10.1111/j.1745-4522.2009.01169.x

## Description

### [Technical Field]

The present disclosure relates to a method of preparing a fatty acid ethyl ester, including: preparing a vegetable lipid; performing transesterification by mixing the vegetable lipid, a first fatty acid ethyl ester, and lipase in a reactor to prepare a second fatty acid ethyl ester; removing the triglycerides and diglycerides present in the reactor; and separating the second fatty acid ethyl ester from the other various fatty acid ethyl ester via rectification from the reactor, wherein the method excludes adding an organic solvent.

### [Background Art]

Fatty acid esters are in a form where fatty acids and alcohols are bonded by an ester linkage. The types of the alcohols that bind to the fatty acids are mainly methanol, ethanol, propanol, butanol, *etc.,* and thus, fatty acid methyl esters, fatty acid ethyl esters, fatty acid propyl esters, fatty acid butyl esters, *etc.,* can be prepared therefrom.

Fatty acid esters have a wide range of applications, but fatty acid esters are utilized most in the field of biodiesel. While exhaust gases emitted in the combustion of hydrocarbons cause environmental problems and are a major cause of global warming, biodiesel has attracted attention as an economical and eco-friendly alternative fuel to solve these environmental problems. Vegetable lipids have a high calorific value to be used as a fuel, but they are disadvantageous in that they have a high viscosity as a polymer material. To solve the problem, fatty acid alkyl esters, which are converted to have a small molecule structure by transesterification with alcohols, are used as biodiesel. Among these, fatty acid methyl esters and fatty acid ethyl esters are mainly used because of low price.

Among the alcohols used in fatty acid esters, methanol and ethanol are economically advantageous. Methanol is mainly obtained from petroleum, but ethanol is obtained from corn, sugar cane, *etc.,* and is more eco-friendly than methanol. Additionally, methanol is known to be harmful to the human body and cause blindness and severe vomiting. However, ethanol is known to be metabolized in the body and converted into acetic acid, which is less toxic to humans than methanol.

Accordingly, fatty acid ethyl esters are preferred in industries where safety is important, such as in the food and cosmetic fields. At present, many fatty acid ethyl esters, such as decanoic acid ethyl ester (decanoic ethyl ester , ethyl decanoate, ethyl caprate, *etc.),* octanoic acid ethyl ester (octanoic ethyl ester, ethyl octanoate, ethyl caprylate, *etc.),* palmitic acid ethyl ester (palmitic ethyl ester, ethyl palmitate, ethyl hexadecanoate, *etc.),* stearic acid ethyl ester (stearic ethyl ester, ethyl stearate, ethyl octadecanoate, *etc.*)*, etc.,* are used in foods as flavoring agents. Additionally, fatty acid ethyl esters may be used as a raw material for fatty acids when modifying fatty acid compositions by enzymatic exchange of an ester as a method for modifying edible fats. Additionally, fatty acid ethyl esters of various compositions are used in cosmetics as flavorings, hair conditioners, skin conditioners (softeners), *etc.* Conventionally, fatty acid ethyl esters are prepared using animal/vegetable lipid such as palm oil, soybean oil, canola oil, fish oil, coconut oil, *etc.,* and free fatty acids derived from animal/vegetable lipid, by transesterification at a high temperature (70°C to 90°C) using an alcohol and a catalyst, and may be obtained by processes for separating glycerin, unreacted fatty acids, unreacted ethanol, *etc.* Transesterification reactions of vegetable oil are also known from EP 0 502 697 and EP 2 561 763.

US 2018/030485 (D2) discloses a process for producing a fat composition rich in triglycerides from raw material fat containing saturated fatty acids. The process includes a step of transesterification by means of a lipase and a rectification step. However, the rectification step is not carried out to separate some specific fatty acid ethyl ester from fatty acid ethyl esters. In addition, the process is carried out in presence of an organic solvent.

Recently, as negative views on environmental problems relating to chemical synthesis methods have spread, methods of producing fatty acid ethyl esters using enzymes are being developed. Nelson *et al.* prepared fatty acid ethyl esters and fatty acid methyl esters from vegetable lipidvegetable lipids and alcohols using lipase expressed in various microorganisms such as Mucor miehei, C. antartica, Pseudomonas cepacia, Rhizopus delemar, Geotrichum candium, etc. (J Am Oil Chem Soc, 73(8): 1191 to 1195, 1996). Additionally, Korean Patent No. 10-1357298 discloses a method of preparing fatty acid ethyl esters by ethanolysis using ethanol in the presence of an enzyme catalyst extracted from at least one microorganism selected from the group consisting of *Candida* species, *Rhizopus* species, *Mucor* species, *Aspergillus* species, and *Pseudomonas* species, during the process of preparing omega-3 polyunsaturated fatty acids.

However, the above method uses an organic solvent such as ethanol and requires an additional step of removing residual ethanol after the enzymatic reaction using an organic solvent such as ethanol, and there is a possibility that safety problems such as fire may occur during the process because the flash point of ethanol is 16°C. Additionally, the method has a disadvantage in that ethanol may remain in the final product, and thus there is a possibility that problems such as skin irritation may occur, particularly in the field of cosmetics. The prior art described above has focused on the preparation of fatty acid ethyl esters using a fatty acid, an animal/vegetable lipid, and a lower alcohol along with a chemical or enzymatic catalyst, and active discussion has not been made with regard to the preparation of fatty acid ethyl esters by the enzymatic method without the use of ethanol. Additionally, after the preparation of the fatty acid ethyl esters from the oil, it is very important to separate only the specific fatty acid ethyl esters with high purity. However, no detailed description is available with regard to the technique for its separation at high purity.

In this regard, the present applicant has made many efforts to develop a fatty acid ethyl ester applicable to foods and cosmetics while simultaneously preparing the fatty acid ethyl ester by enzymatic transesterification of specific fatty acids contained in vegetable lipids without using an organic solvent such as ethanol, thereby completing the present disclosure.

### [Disclosure]

### [Technical Problem]

An object of the present disclosure is to provide a method of preparing a fatty acid ethyl ester as recited in claim 1.

### [Technical Solution]

The present applicant has made many efforts to develop a fatty acid ethyl ester applicable to foods and cosmetics while simultaneously preparing the fatty acid ethyl ester by enzymatic transesterification of specific fatty acids contained in vegetable lipids without using an organic solvent such as ethanol, thereby completing the present disclosure.

### [Advantageous Effects of the Invention]

The method for preparing a fatty acid ethyl ester of the present disclosure can preclude the use of chemicals which may be harmful to the human body or can cause irritation. Accordingly, the fatty acid ethyl ester thus prepared can be widely used for food compositions or cosmetic compositions.

### [Best Mode for Carrying Out the Invention]

In order to achieve the above objects, there is provided a method of preparing a fatty acid ethyl ester, including: preparing a vegetable lipid; performing transesterification by mixing the vegetable lipid, a first fatty acid ethyl ester, and lipase in a reactor to prepare a second fatty acid ethyl ester; removing the triglycerides and diglycerides present in the reactor; and separating the second fatty acid ethyl ester from the other various fatty acid ethyl ester via rectification from the reactor, wherein the method excludes adding an organic solvent.

A food composition containing a fatty acid ethyl ester may be prepared by the above method. A cosmetic composition containing a fatty acid ethyl ester may be prepared by the above method.

Hereinafter, the present disclosure is explained in detail.

The vegetable lipid may contain the vegetable lipid itself or a fraction thereof. Non-limiting examples of the vegetable lipid that may be used in the present disclosure may include canola oil, coconut oil, palm kernel oil, palm oil, canola oil, sunflower oil, soy bean oil, cotton seed oil, rice bran oil, corn oil, olive oil, shea fat, mango kernel fat, illippe butter (Borneo tallow, Shorea stenoptera, or Pentadema butyracea), sal oil (Sherea robusta), kokum oil (Garcinia indica), but the vegetable lipid is not limited thereto and any vegetable lipid used in the art may be included. Preferably, the vegetable lipid may be palm kernel oil, palm oil, and sunflower oil, but is not limited thereto.

Alternatively, the vegetable lipid may be fractionated to obtain a fat fraction containing 1,3-dipalmitoyl-2-oleoylglycerol (POP) for use as the vegetable lipid. The fractionation may be performed by dry fractionation or solvent fractionation, but is not limited thereto, and any method that can provide a POP-containing oil with a difference in the content of saturated fatty acids and unsaturated fatty acids from the raw materials for the vegetable lipid known in the art may be used without limitation. For example, the fractionation method may be selectively used depending on the characteristics of the raw materials for the vegetable lipid to be used. In the case of solvent fractionation, an organic solvent capable of dissolving the raw materials for the vegetable lipid, such as hexane, acetone, methyl ethyl ketone, ethanol, *etc.,* can be used without limitation. The vegetable lipid to be used in the present disclosure may be an oil, which contains 35 wt% to 70 wt% of 1,3-dipalmitoyl-2-oleoylglycerol (POP) and has an iodine value of 35 to 55.

The method for preparing a fatty acid ethyl ester includes (b) transesterification by mixing the vegetable lipid, a first fatty acid ethyl ester, and lipase within a reactor to prepare a second fatty acid ethyl ester. The transesterification performed in step (b) may be represented by the following reaction scheme.

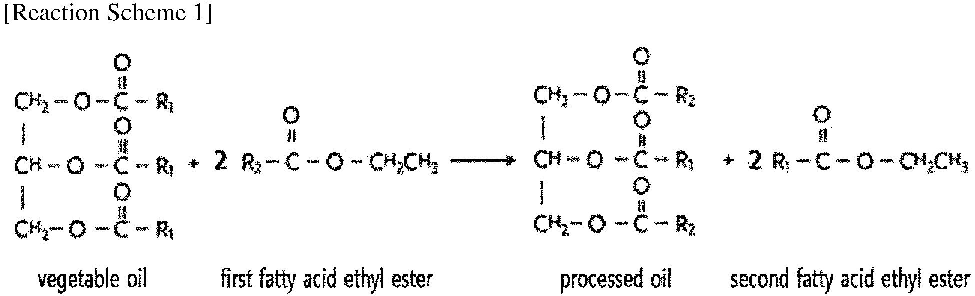

The lipase that can be used in the enzymatic transesterification reaction may be a lipase derived from *Mucor miehei* or *Thermomyces lanuginosus,* but the origin thereof is not limited thereto, and any lipase known in the art can be used. As shown in the above reaction scheme, the lipase to be used in the present disclosure is a material serving as a catalyst for preparing a second fatty acid ethyl ester where R₁ is substituted, by exchanging R₁ substituted in the triglyceride of the vegetable lipid with R₂ substituted in the first fatty acid ethyl ester. More specifically, the lipase may be one which has specificity at sn-1,3 positions or one which does not have specificity. The reaction with the enzyme may be performed at 30°C to 60°C for 1 hour to 30 hours, but the reaction conditions are not limited thereto.

According to an embodiment, the first fatty acid ethyl ester may be at least one fatty acid ethyl ester selected from the group consisting of a stearic acid ethyl ester, an oleic acid ethyl ester, a linoleic acid ethyl ester, a palmitic acid ethyl ester, a caprylic acid ethyl ester, and a capric acid ethyl ester.

Meanwhile, according to an embodiment, the molar ratio between the vegetable lipid and the first fatty acid ethyl ester in step (b) may be 2:1 to 1:5.

Meanwhile, according to an embodiment, the second fatty acid ethyl ester may be at least one fatty acid ethyl ester selected from the group consisting of a stearic acid ethyl ester, an oleic acid ethyl ester, and a palmitic acid ethyl ester.

The method of preparing a fatty acid ethyl ester includes (c) removing the triglycerides and diglycerides present in the reactor.

The reactants obtained by transesterification in step (b) may also contain triglycerides and diglycerides, in addition to the fatty acid ethyl ester. Accordingly, it is necessary to remove these triglycerides and diglycerides so as to obtain fatty acid ethyl esters with high purity. For example, fatty acid ethyl esters can be obtained by removing these triglycerides and diglycerides by performing simple distillation or distillation in a distillation column with 5 stages or fewer under the conditions of 100°C to 250°C and a degree of vacuum 1 mbar to 3 mbar, and thereby a fatty acid ethyl ester can be obtained. By removal of the triglycerides and diglycerides from fatty acid ethyl esters, these can be further utilized as a processed oil, which is advantageous from the economical aspect.

Step (c) may be performed by simple distillation or in a distillation column with 5 stages or fewer under the conditions of 220°C to 245°C, a degree of vacuum 1 mbar to 3 mbar, and a reflux ratio of 1 to 3. The degree of vacuum, reflux ratio, and temperature can be adjusted within the above ranges, and once one of the values is adjusted, other values can be adjusted as well.

The method of the present disclosure includes (d) separating a second fatty acid ethyl ester via rectification from the reactor. The content ratio of the fatty acid ethyl ester can be increased by removing triglycerides and diglycerides in step (c). However, in the product obtained through step (c), various fatty acid ethyl esters may be mixed depending on the kind of various fatty acids contained in the raw material. Accordingly, it is required to selectively rectify only the fatty acid ethyl ester to be separated and purified at high purity. The rectification refers to a method of precisely separating volatile materials with a similar boiling point through repeated reflux. More specifically, since the use of equilibrium distillation for separating components with similar volatility results in poor purity of the condensed vapor and remaining liquid, the generated vapor is brought into contact with a part of the condensate returned to the distiller and countercurrent, and the components having a low boiling points are evaporated again using the condensation heat of the vapor. The process of separating and concentrating materials more precisely by repeating this process is called rectification, and the simple distillation can be connected in series and the vapor can be concentrated while the steam is in contact with the reflux. For example, the product having been subjected to step (c) may be rectified in a distillation column with 15 to 30 stages, at a degree of vacuum of 1 mbar to 3 mbar, a temperature of 100°C to 250°C, and a reflux ratio of 1 to 5, thereby separating only the desired fatty acid ethyl ester. However, the detailed conditions of the rectification are not limited to those described above, and a more detailed degree of vacuum, temperature, reflux ratio, *etc.* can be controlled depending according to the kind of fatty acid ethyl ester to be separated. The fatty acid ethyl ester having a purity of 92% to 100%; specifically, a purity of 95% or more; and more specifically, a purity of 98% or more can be obtained through the rectification process.

Step (d) may be performed by simple distillation or in a distillation column with 15 stages or fewer under the conditions of 205°C to 240°C, a degree of vacuum 1 mbar to 3 mbar, and a reflux ratio of 1 to 3. The degree of vacuum, reflux ratio, and temperature can be adjusted within the above ranges, and once one of the values is adjusted, other values can be adjusted as well.

The method for preparing a fatty acid ethyl ester is by an ester exchange reaction using an enzyme, and the method is characterized in that the method does not include adding an organic solvent. In conventional reactions using a chemical catalyst, it is common to use an organic solvent. However, since the material used as a solvent cannot be completely removed from the fatty acid ethyl ester, which is a subsequent product, it may be irritating or harmful if it is applied to the human body as a food composition or cosmetic composition. Therefore, the preparation method of the present disclosure does not use an organic solvent, *etc.,* which may be harmful to the human body, and thus the material prepared thereby is advantageous for use in a food composition or cosmetic composition.

A food composition containing a fatty acid ethyl ester prepared by the above method can be provided. The fatty acid ethyl ester can be used for a food composition for the purpose of enhancing edible texture, or as an additive such as a flavoring agent, *etc.*

Examples of the foods in which a fatty acid ethyl ester is contained may include meats, sausages, breads, chocolates, candies, snacks, cookies, pizzas, ramens, other noodles, gums, dairy products including ice cream, various kinds of soups, beverages, teas, drinks, alcoholic beverages, vitamin complexes, *etc.,* and may include all kinds of foods from the conventional point of view.

Furthermore, the foods may be prepared as a health functional food used for the purpose of a health supplement, by adding an active ingredient known to exert a biological regulation function in the living body such as biodefense, regulation of biorhythm, prevention and recovery of diseases, *etc.*

Furthermore, the present disclosure describes a cosmetic composition containing the fatty acid ethyl ester prepared by the method described above.

The cosmetic composition may be prepared into a formulation selected from the group consisting of a solution, an ointment for external use, a cream, a foam, a nutrition emollient, a soft emollient, a pack, a soft water, an emulsion, a makeup base, an essence, lip balm, a soap, a liquid cleansing agent, a bath preparation, a sunscreen cream, a sun oil, a suspension, a paste, a gel, a lotion, powders, a surfactant-containing cleansing agent, an oil, a powder foundation, an emulsion foundation, a wax foundation, a patch, and a spray, but the formulation is not limited thereto. Specifically, in the cosmetic composition, the fatty acid ethyl ester prepared by the above method may be used as an additive (*e.g.,* a solubilizing agent, an emulsifier, a dispersing agent, a flavoring agent, a softening agent, *etc.*) for improving or stabilizing the solubility of other active ingredients.

Additionally, the cosmetic composition of the present disclosure may further contain at least one kind of a cosmetically acceptable carrier to be mixed into a common cosmetic composition for the skin, and for example, commonly used ingredients such as a fat content, water, a surfactant, a moisturizer, a lower alcohol, a thickener, a chelating agent, a pigment, a preservative, a perfume, *etc.,* may be appropriately mixed therein, but the additional ingredients are not limited thereto.

Alternatively, the cosmetic composition may be prepared into functional cosmetics by further containing a component having an antioxidant effect, a wrinkle-improving effect, an anti-aging effect, and/or a UV-blocking effect. For the ingredient having the antioxidant effect, wrinkle-improving effect, anti-aging effect, and/or UV-blocking effect, any active ingredient being used in functional cosmetics known in the art may be used without limitation.

The present disclosure provides a method for preparing a fatty acid ethyl ester by enzymatic transesterification without using an organic solvent or chemical catalyst. The fatty acid ethyl ester thus prepared can preclude the possibility that it may contain chemical residues which are harmful to the human body or can cause irritation. Accordingly, the fatty acid ethyl ester thus prepared can be used for food compositions or cosmetic compositions.

### [Mode for Carrying Out the Invention]

Hereinafter, the present disclosure will be described in detail through specific examples. However, the description proposed herein is merely a preferred example for the purpose of illustrations only, not intended to limit the scope of the present disclosure, and therefore it should be understood that the examples are provided for a better explanation to an ordinary person skilled in the art.

### Example 1: Preparation of fatty acid ethyl ester by enzymatic transesterification

In this Example, palmitic acid ethyl esters were prepared by the method described below. For the vegetable lipid used as a raw material, palm oil-derived fat fraction containing high palmitic acid content was selected. In particular, the palm oil-derived fat fraction had a POP content of 35% to 70% and an iodine value of 35 to 55. For the fatty acid ethyl ester used for the exchange of the fatty acid and supply of an ethyl group along with the palm oil-derived fat fraction, a stearic acid ethyl ester was used.

Specifically, the palm oil-derived fat fraction and stearic acid ethyl ester were mixed in a molar ratio of 1:0.5 to 1:5. The mixture was mixed with 5 wt% of lipase derived from *Mucor miehei* and the transesterification was performed in a reactor equipped with a stirrer (stirring transesterification). Meanwhile, apart from the above, a glass column of double jacket type was filled with an enzyme and the transesterification was performed while continuously passing the raw material mixture through the column (continuous transesterification). In particular, the reaction temperature was constantly maintained at 50°C. Ethyl palmitic acid ethyl ester produced according to the reaction time was collected.

### Example 2: Separation of triglycerides

Distillation was performed so as to separate triglycerides from the stearic acid ethyl ester and the palmitic acid ethyl ester obtained by transesterification in Example 1.

**[Table 1]**

| Temperature of Bottom of Column (Degree of Vacuum; 2 mbar / Reflux Ratio; 1) | | Triglyceri des Diglycerid es (%) | Fatty Acid Ethyl Ester (%) | Free Fatty Acid (%) |
|---|---|---|---|---|
| 220° C | Before Distillation (Feed) | 55.8 | 42.6 | 1 |
| | After Distillation (Top) | 0 | 97.2 | 1.2 |
| | After Distillation (Bottom) | 93.3 | 5.3 | 0.5 |
| 235° C | Before Distillation (Feed) | 55.8 | 42.6 | 1 |
| | After Distillation (Top) | 0 | 99 | 0.1 |
| | After Distillation | 96.8 | 2.4 | 1.1 |
| | (Bottom) | | | |
| 240° C | Before Distillation (Feed) | 55.8 | 42.6 | 1 |
| | After Distillation (Top) | 0.2 | 97.8 | 1.3 |
| | After Distillation (Bottom) | 97.2 | 1.2 | 0.6 |
| 245° C | Before Distillation (Feed) | 55.8 | 42.6 | 1 |
| | After Distillation (Top) | 0.7 | 96.5 | 1.8 |
| | After Distillation (Bottom) | 97.8 | 1.1 | 0.4 |

Distillation was performed in a 4-stage distillation column under the conditions of a degree of vacuum of 2 mbar, a temperature of 220°C to 245°C at the bottom of the column, and a reflux ratio of 1 to remove the triglycerides, and thereby stearic acid ethyl esters and palmitic acid ethyl esters (*i.e.,* fatty acid ethyl esters) were obtained.

### Example 3: Analysis of compositions and contents of fatty acid ethyl esters

### 3.1. GC-FID Analysis

The compositions and contents of fatty acid ethyl esters obtained from Example 2 were analyzed by gas chromatography (GC). Specific analytical conditions are shown in Table 2 below. Prior to analysis, fatty acid ethyl esters may be methylated by a conventional method for the analysis of fatty acids and 50 µL of the obtained fatty acid ethyl esters may be dissolved in 1 mL of hexane for use. In this example, the analysis was performed while omitting the methylation process.

**[Table 2]**

| Device Name | Agilent, 1200 HPLC Chemstation |
|---|---|
| Column | SP^{™}-2560 Capillary GC Column (L × I. D. 100 m × 0.25 mm, df 0.20 µm) |
| Type of Detector | Flame Ionization Detector (FID) |
| Amount of Injection | 1 µL |
| Inlet Conditions | Heater: 250°C |
| | Pressure: 48 psi |
| | Septum Purge Flow: 3 mL/min |
| | Split Ratio: 50:1 |
| Column Conditions | Flow: 1.5 mL/min |
| | Temperature: 140°C, 5 min |
| | 4°C/min, 210°C, 10 min |
| | 4°C/min, 240°C, 5 min |
| | 4°C/min, 250°C, 5 min |
| Detector Conditions | Heater: 250°C |
| | H₂ Flow: 35 mL/min |
| | Air Flow: 300 mL/min |
| | Makeup Flow (He): 20 mL/min |

### 3.2. Analysis of composition of fatty acid ethyl esters

The compositions and contents of the fatty acid ethyl esters obtained by the distillation process according to Example 2 were analyzed. The compositions of the fatty acid ethyl esters confirmed by GC-FID are shown in Table 3 below.

**[Table 3]**

| Ratio of Substrates (Palm Oil-Derived Fat Fraction: Stearic Acid Ethyl Ester) | Reaction Time | Stearic Acid Ethyl Ester (%) | Oleic Acid/Linoleic Acid Ethyl Ester (%) | Palmitic Acid Ethyl Ester (%) |
|---|---|---|---|---|
| 1:0.5 | 8 Hr | 66.1 | 3.1 | 30.8 |
| 1:1 | 8 Hr | 60.4 | 3.6 | 35.4 |
| 1:2 | 8 Hr | 50.6 | 5.7 | 43.5 |
| 1:3 | 8 Hr | 48.3 | 7.9 | 43.2 |
| 1:4 | 8 Hr | 47.8 | 8.0 | 43.8 |
| 1:5 | 8 Hr | 49.3 | 8.1 | 42.1 |

As shown in Table 3 above, as the molar ratio of stearic acid ethyl esters to palm oil-derived fat fraction was increased, the amount of palmitic acid ethyl esters was increased based on the same reaction time. However, even when the molar ratio of stearic acid ethyl esters to palm oil-derived fat fraction was increased from 1:2 to 1:5, there was no great difference in terms of the amount of the palmitic acid ethyl esters. Accordingly, the most appropriate ratio was 1:2 from the aspect of process efficiency, in which the content of the palmitic acid ethyl esters was 43.5%.

### Example 4: Improvement of purity of palmitic acid ethyl esters

Rectification was performed to improve the purity of palmitic acid ethyl esters (the product) by separating stearic acid ethyl esters (the raw material) from the fatty acid ethyl ester mixture obtained from Example 2. Specifically, distillation was performed in a 22-stage distillation column under the conditions of a degree of vacuum of 2 mbar, a temperature of 235°C at the bottom of the column, and a reflux ratio of 3, and thereby high-purity palmitic acid ethyl esters were obtained.

### Example 5: Analysis of purity of purified palmitic acid ethyl esters

The purity of palmitic acid ethyl esters obtained by rectification according to Example 4 was analyzed. This analysis was performed using GC-FID as in Example 3 and the results are shown in Table 4 below.

**[Table 4]**

| Temperature of Bottom of Column (Degree of Vacuum; 2 mbar / Reflux Ratio; 3) | Time-point and Position of Measurement | Stearic Acid Ethyl Ester (%) | Oleic Acid/Linoleic Acid Ethyl Ester (%) | Palmitic Acid Ethyl Ester (%) |
|---|---|---|---|---|
| 205°C | Before Distillation (Feed) | 50.6 | 5.7 | 43.5 |
| | After Distillation (Top) | 2.4 | 0.5 | 92. |
| | After Distillation (Bottom) | 84 | 10.8 | 3.5 |
| 220°C | Before Distillation (Feed) | 50.6 | 5.7 | 43.5 |
| | After Distillation (Top) | 1.3 | 0.2 | 95.4 |
| | After Distillation (Bottom) | 87.3 | 9.2 | 1.9 |
| 235°C | Before Distillation (Feed) | 50.6 | 5.7 | 43.5 |
| | After Distillation (Top) | 0.8 | 0.0 | 98.6 |
| | After Distillation (Bottom) | 89.6 | 8.5 | 1.3 |
| 240°C | Before Distillation (Feed) | 50.6 | 5.7 | 43.5 |
| | After Distillation (Top) | 1.8 | 0.4 | 96.2 |
| | After Distillation (Bottom) | 86.9 | 9.5 | 2.0 |

As shown in Table 4 above, it was confirmed that the purity of palmitic acid ethyl esters was increased to 98.6% by an additional distillation process. Stearic acid ethyl esters produced from the bottom of a distillation column after distillation can have a purity of 98% or higher, and thus the stearic acid ethyl esters can be reused as a raw material for fatty acid ethyl esters in the enzymatic reaction of Example 1.

### Example 6: Evaluation of diversity of kinds of fatty acid ethyl esters

Fatty acid ethyl esters were prepared by enzymatic transesterification, similar to that of Example 1, using high oleic sunflower oil, canola oil, and coconut oil as raw materials instead of palm mid fraction (PMF) fat based on the fact that various fatty acid compositions can be contained according to the kinds of vegetable lipids, in addition to the palmitic acid ethyl esters prepared using the palm mid fraction (PMF) fat as a vegetable lipid according to Example 1. The three kinds of vegetable lipids and stearic acid ethyl esters were mixed in a molar ratio of 1:2 and 5 wt% of lipase derived from *Mucor miehei* was added thereto and allowed to react in a reactor equipped with a stirrer for 12 hours. The reaction temperature was maintained at 45°C.

Triglycerides were removed from the mixture obtained by the esterification using the same method as in Example 2 to remove triglyceride and the fatty acid ethyl esters were separated. The compositions and contents of the separated fatty acid ethyl esters were analyzed using a method similar to that of Example 3 (GC-FID) and the results are shown in Table 5 below.

**[Table 5]**

| | Kinds of Vegetable lipids | | |
|---|---|---|---|
| | High Oleic Sunflower Oil (%) | Canola Oil (%) | Coconut Oil (%) |
| Caprylic Acid/Capric Acid Ethyl Ester | - | - | 7.1 |
| Lauric Acid Ethyl Ester | - | - | 23.3 |
| Stearic Acid Ethyl Ester | 50.3 | 52.5 | 50.3 |
| Oleic Acid Ethyl Ester | 39.8 | 29.4 | 2.7 |
| Linoleic Acid Ethyl Ester | 4.2 | 9.6 | - |
| Linolenic Acid Ethyl Ester | - | 4.7 | - |

As shown in Table 5 above, oleic acid ethyl esters were prepared from high oleic sunflower oil; oleic acid ethyl esters and linolenic acid ethyl esters from canola oil; and caprylic acid and capric acid ethyl esters from coconut oil, respectively. The purity of the fatty acid ethyl esters thus obtained can be improved by rectification using a distillation column similar to that of Example 4.

## Claims

1. A method of preparing a fatty acid ethyl ester, comprising:
(a) preparing a vegetable lipid;
(b) performing transesterification by mixing the vegetable lipid, a first fatty acid ethyl ester, and lipase in a reactor to prepare a second fatty acid ethyl ester;
(c) removing the triglycerides and diglycerides present in the reactor; and
(d) separating the second fatty acid ethyl ester from the other various fatty acid ethyl ester via rectification from the reactor,
wherein the method excludes adding an organic solvent.

2. The method according to claim 1, wherein the molar ratio between the vegetable lipid and the first fatty acid ethyl ester in step (b) is 2:1 to 1:5.

3. The method according to claim 1, wherein step (c) is performed in a distillation column under the conditions of 220°C to 245°C, a degree of vacuum 1 mbar to 3 mbar, and a reflux ratio of 1 to 3.

4. The method according to claim 1, wherein step (d) is performed in a distillation column under the conditions of 205°C to 240°C, a degree of vacuum 1 mbar to 3 mbar, and a reflux ratio of 1 to 3.

5. The method according to claim 1, wherein the vegetable lipid is at least one selected from the group consisting of palm oil, sunflower oil, canola oil, and coconut oil, and the vegetable lipid comprises 35 wt% to 70 wt% of 1,3-dipalmitoyl-2-oleoylglycerol (POP) and has an iodine value of 35 to 55.

6. The method according to claim 1, wherein the first fatty acid ethyl ester is at least one fatty acid ethyl ester selected from the group consisting of a stearic acid ethyl ester, an oleic acid ethyl ester, a linoleic acid ethyl ester, a palmitic acid ethyl ester, a caprylic acid ethyl ester, and a capric acid ethyl ester.

7. The method according to claim 1, wherein the second fatty acid ethyl ester is at least one fatty acid ethyl ester selected from the group consisting of a stearic acid ethyl ester, an oleic acid ethyl ester, and a palmitic acid ethyl ester.

## Patentansprüche

1. Verfahren zur Herstellung eines Fettsäureethylesters, umfassend:
(a) Herstellen eines pflanzlichen Lipids;
(b) Durchführen einer Transveresterung durch Mischen des pflanzlichen Lipids, eines ersten Fettsäureethylesters und einer Lipase in einem Reaktor, um ein zweites Fettsäureethylester herzustellen;
(c) Entfernen der Triglyceride und Diglyceride, die in dem Reaktor vorhanden sind; und
(d) Trennen des zweiten Fettsäureethylesters von den anderen verschiedenen Fettsäureethylestern mittels Gegenstromdestillation aus dem Reaktor,
wobei das Verfahren ein Zugeben eines organischen Lösungsmittels ausschließt.

2. Verfahren nach Anspruch 1, wobei das Molverhältnis zwischen dem pflanzlichen Lipid und dem ersten Fettsäureethylester in Schritt (b) 2:1 bis 1:5 beträgt.

3. Verfahren nach Anspruch 1, wobei Schritt (c) in einer Destillationskolonne unter den Bedingungen von 220°C bis 245°C, einem Vakuumgrad von 1 mbar bis 3 mbar und einem Rückflussverhältnis von 1 bis 3 durchgeführt wird.

4. Verfahren nach Anspruch 1, wobei Schritt (d) in einer Destillationskolonne unter den Bedingungen von 205°C bis 240°C, einem Vakuumgrad von 1 mbar bis 3 mbar und einem Rückflussverhältnis von 1 bis 3 durchgeführt wird.

5. Verfahren nach Anspruch 1, wobei das pflanzliche Lipid mindestens eines ist, das aus der Gruppe bestehend aus Palmöl, Sonnenblumenöl, Rapsöl und Kokosöl ausgewählt ist, und
das pflanzliche Lipid 35 Gew.-% bis 70 Gew.-% 1,3-Dipalmitoyl-2-oleoylglycerin (POP) umfasst und eine Iodzahl von 35 bis 55 aufweist.

6. Verfahren nach Anspruch 1, wobei der erste Fettsäureethylester mindestens ein Fettsäureethylester ist, der aus der Gruppe bestehend aus einem Stearinsäureethylester, einem Ölsäureethylester, einem Linolsäureethylester, einem Palmitinsäureethylester, einem Caprylsäureethylester und einem Caprinsäureethylester ausgewählt ist.

7. Verfahren nach Anspruch 1, wobei der zweite Fettsäureethylester mindestens ein Fettsäureethylester ist, der aus der Gruppe bestehend aus einem Stearinsäureethylester, einem Ölsäureethylester und einem Palmitinsäureethylester ausgewählt ist.

## Revendications

1. Procédé de préparation d'un ester éthylique d'acide gras, comprenant :
(a) la préparation d'un lipide végétal ;
(b) la mise en œuvre d'une trans-estérification par mélange du lipide végétal, d'un premier ester éthylique d'acide gras, et d'une lipase dans un réacteur pour préparer un deuxième ester éthylique d'acide gras ;
(c) l'élimination des triglycérides et diglycérides présents dans le réacteur ; et
(d) la séparation du deuxième ester éthylique d'acide gras de l'autre divers ester éthylique d'acide gras via rectification depuis le réacteur,
dans lequel le procédé exclut l'addition d'un solvant organique.

2. Procédé selon la revendication 1, dans lequel le rapport molaire entre le lipide végétal et le premier ester éthylique d'acide gras dans l'étape (b) est de 2/1 à 1/5.

3. Procédé selon la revendication 1, dans lequel l'étape (c) est effectuée dans une colonne de distillation dans des conditions comprenant une température de 220 °C à 245 °C, un degré de vide de 1 mbar à 3 mbar, et un taux de reflux de 1 à 3.

4. Procédé selon la revendication 1, dans lequel l'étape (d) est effectuée dans une colonne de distillation dans des conditions comprenant une température de 205 °C à 240 °C, un degré de vide de 1 mbar à 3 mbar, et un taux de reflux de 1 à 3.

5. Procédé selon la revendication 1, dans lequel le lipide végétal est au moins l'un choisi dans le groupe constitué par l'huile de palme, l'huile de tournesol, l'huile de canola, et l'huile de coco, et
le lipide végétal comprend 35 % en poids à 70 % en poids de 1,3-dipalmitoyl-2-oléoylglycérol (POP) et a un indice d'iode de 35 à 55.

6. Procédé selon la revendication 1, dans lequel le premier ester éthylique d'acide gras est au moins un ester éthylique d'acide gras choisi dans le groupe constitué par un ester éthylique d'acide stéarique, un ester éthylique d'acide oléique, un ester éthylique d'acide linoléique, un ester éthylique d'acide palmitique, un ester éthylique d'acide caprylique, et un ester éthylique d'acide caprique.

7. Procédé selon la revendication 1, dans lequel le deuxième ester éthylique d'acide gras est au moins un ester éthylique d'acide gras choisi dans le groupe constitué par un ester éthylique d'acide stéarique, un ester éthylique d'acide oléique, et un ester éthylique d'acide palmitique.
